# EUROPÄISCHE PATENTSCHRIFT

(11) **EP 0 837 662 B1**
(45) Veröffentlichungstag und Bekanntmachung des Hinweises auf die Patenterteilung: **04.04.2001**
(21) Anmeldenummer: 96923962.3
(22) Anmeldetag: 26.06.1996
(51) Int. Cl.: A61F 13/14

(54) **BÜSTENHALTEREINLAGE**
INSERT LINING FOR A BRASSIERE
PIECE RAPPORTEE POUR SOUTIEN-GORGE

(30) Priorität: 30.06.1995 DE 19523920
(43) Veröffentlichungstag der Anmeldung: 29.04.1998
(73) Patentinhaber: Umwelt-Technics-Nord GmbH, D-22851 Norderstedt (DE)
(72) Erfinder: GRONHOLZ, Claus, D-22851 Norderstedt (DE)
(74) Vertreter: Graalfs, Edo, Dipl.-Ing. Patentanwälte Hauck, Graalfs, Wehnert, Döring, Siemons
(86) Internationale Anmeldenummer: EP9602820
(87) Internationale Veröffentlichungsnummer: WO9701994

(56) Entgegenhaltungen:
- CH-A- 678 597
- DE-C- 836 238
- FR-A- 1 574 347
- US-A- 3 738 362

## Beschreibung

Die Erfindung bezieht sich auf einen Büsterhalter nach dem Oberbegriff des Patentanspruches 1.

Zur Pflege der weiblichen Brust gibt es Wirkstoffpräparate, beispielsweise Salben oder Flüssigkeiten, die auf die Haut des Busens aufgetragen werden, um über einen längeren Zeitraum einzuwirken. Bei der direkten Auftragung auf die Haut ist eine Dosierung der Wirkstoffpräparate nur schwer bzw. recht umständlich möglich, beispielsweise durch wiederholtes Auftragen geringer Mengen. Eine über einen längeren Zeitraum gleichmäßige Dosierung kann auf diese Weise nur in gewissen Grenzen erreicht werden.

Es sind Einlagen für Büstenhalter bekannt, die verschiedenen Zwecken dienen. So gibt es z.B. Stilleinlagen (siehe G 81 19 845.0, DE 32 06 371 A1 oder G 83 15 723.9) und Formeinlagen (siehe DE 31 20 875 C2).

Aus CH-A-678 597 ist ein Verband zur Versorgung eines Körperbezirks bekannt geworden, der auch als Stillkompresse dienen kann. Die Kompresse weist eine Haube auf, deren Form an die Form der weiblichen Brust angepaßt ist. Im Inneren der Haube ist eine saugfähige Einlage vorgesehen, die zur Behandlung der Brust bestimmte Flüssigkeit oder aus der Brust ausfließende Flüssigkeit aufnehmen kann. An der Kante der Haubenmündung ist eine Breitfläche vorgesehen mit einer Klebelippe zur Anbringung der Haube an der weiblichen Brust.

Der Erfindung liegt die Aufgabe zugrunde, eine Einlage für einen Büstenhalter zu schaffen, über die Wirkstoffe zur Pflege der weiblichen Brust oder zu deren therapeutischen Behandlung auf bequeme Weise und mit konstanter Dosierung mit der Haut in Kontakt gebracht werden können.

Die Aufgabe wird durch die Merkmale des Patentanspruchs 1 gelöst.

Vorteile der Einlage sind, daß die Wirkstoffzonen als Reservoir für Wirkstoffpräparate dienen und gleichzeitig einen oder mehrere Wirkstoffe an die Haut abgeben, wobei die Abgabemenge durch die Beschaffenheit der Wirkstoffzonen, wie unten beschrieben wird, mitbestimmt wird. Zudem dient die Kompensationszone der Einlage als atmungsaktiver Bereich, insbesondere zur Vermeidung von Schweißbildung. Es ist jedoch auch ohne weiteres möglich, daß die Wirkstoffzone zugleich ein atmungsaktiver Bereich ist.

Die Einlage weist eine eigene Formstabilität auf, so daß sie, ohne mit einem Büstenhalter verbunden zu sein, eine Cupform aufweist.

Die Einlage bedeckt günstigerweise im wesentlichen die gesamte Innenfläche des Cups. Um die Wirkung der Einlage zu maximieren, ist die Größe der Wirkstoff- und der Kompensationszonen so gewählt, daß ihre Gesamtfläche im wesentlichen der Gesamtfläche der Einlage entspricht. In einer vorteilhaften Ausgestaltung sind eine Vielzahl von Wirkstoffzonen und eine Vielzahl von Kompensationszonen vorgesehen, die abwechselnd angeordnet sind. Dabei können die Zonen der einen Art voneinander getrennt, inselartig innerhalb einer Zone der anderen Art angeordnet sein. Günstig ist, wenn die Zonen streifenförmig in Umfangsrichtung oder Längsrichtung verlaufen. Wenn Zonen inselartig innerhalb einer anderen Zone angeordnet sind, kann eine runde Umfangsform der inselartig angeordneten Zonen vorteilhaft sein.

Gemäß einer weiteren vorteilhaften Ausgestaltung weist die Einlage mehrere Lagen auf, von denen eine die Wirkstoff - und die Kompensationszonen enthält. An diese Lage kann eine weitere, weiter außen liegende Lage anschließen, die atmungsaktiv und zumindest in an die Wirkstoffzonen anliegenden Bereichen flüssigkeitsundurchlässig ist. Dadurch wird erreicht, daß das in den Wirkstoffzonen enthaltene in der Regel flüssige oder zähflüssige Wirkstoffpräparat oder die Trägersubstanz, in der die Wirkstoffe enthalten sind, nicht in den Büstenhalter austreten kann. Nach innen kann sich an die speichernde Lage eine weitere Lage anschließen, die zur Übertragung der Wirkstoff und auch zur Übertragung der von den Kompensationszonen aufzunehmenden Körpersubstanzen dienen kann. Bei Verwendung einer solchen Übertragungslage kommt diese anstelle der speichernden Lage in direkten Kontakt mit der Haut. Auch die Übertragungslage kann verschiedene Zonen aufweisen, die mit den Wirkstoffzonen bzw. den Kompensationszonen deckungsgleich sind und diesen entsprechende unterschiedliche Übertragungseigenschaften aufweisen. Damit ein Wirkstoffpräparat, das schon bei der Herstellung der Einlage in diese eingegeben worden ist, nicht schon vor der Benutzung der Einlage auf dieser austreten oder an Wirkfähigkeit verlieren kann, kann als innerste Lage ein abziehbarer Schutzfilm, der luftdicht ist, vorgesehen sein. Alternativ kann die gesamte Einlage nach ihrer Herstellung durch eine umschließende Folie versiegelt werden. Es kann auch vorgesehen sein, daß die Wirkstoffzonen nicht schon bei der Herstellung der Einlage mit einem Wirkstoffpräparat versehen werden, sondern daß die Wirkstoffzonen erst von der Benutzerin mit einem Wirkstoffpräparat versehen werden, beispielsweise durch Einträufeln. Darüber hinaus kann auch vorgesehen sein, daß die Benutzerin nach Benutzung der Einlage Wirkstoffpräparate in die Wirkstoffzonen nachfüllt.

Insbesondere wenn die Gesamtflächen der Wirkstoffzonen und der Kompensationszonen maximal sein sollen, können die verschiedenen Zonen unmittelbar aneinandergrenzen. Vorzugsweise sind dann die Grenzflächen zwischen den Zonen so beschaffen, daß sie eine begrenzte Durchlässigkeit aufweisen. Für das Wirkstoffpräparat und die Trägersubstanz sollte praktisch keine Durchlässigkeit gegeben sein. Wenn die Wirkstoff- und die Kompensationszonen nicht unmittelbar aneinandergrenzen, können zwischen diesen Zonen weitere Zonen liegen, die "neutral" sind. Vorzugsweise weisen diese "neutralen" Zonen, ähnlich wie die Grenzflächen zwischen unmittelbar aneinandergrenzenden Wirkstoff- und Kompensationszonen, praktisch keine Durchlässigkeit für das Wirkstoffpräparat und die Trägerflüssigkeit auf.

Damit sich die Einlage gut an eine Form eines Büstenhaltercups anpassen kann, ist die Einlage vorzugsweise aus flexiblem Material hergestellt. An der Innenseite kann die Einlage thermoplastisch wirken, um bei Hautoberflächentemperatur einen innigen Kontakt zu ermöglichen. Die Einlage kann ein Flachstreifen sein, der aufgerollt und mit einem selbstklebenden Verschluß verschlossen ist. Der Flachstreifen kann so vorgeformt sein, daß er beim Aufrollen eine konische Form erhält. Zusätzlich oder alternativ kann das Material des Flachstreifens so weich sein, daß sich der aufgerollte Flachstreifen sehr leicht der Cupform anpaßt. Dieser Flachstreifen kann Schwächungslinien aufweisen, entlang derer ein Teil des Flachstreifens zu dessen Kürzung abgetrennt werden kann, um die Größe der Einlage an den Cup anzupassen. Die Einlage kann, sowohl wenn sie ein aufgerollter Flachstreifen ist, als auch wenn sie von vornherein in Cupform hergestellt ist, in Längsrichtung eine Trennlinie aufweisen, wobei sich zwei Seitenbereiche entlang der Linie zur Größenveränderung der Einlage einstellbar überlappen. Eine Anpassung der Einlage an Büstenhaltercup verschiedener Größe kann auch dadurch erreicht werden, daß die Einlage in Längsrichtung Ausnehmungen aufweist, die sich von der größten Umfangslinie erstrecken, so daß sich zwischen diesen befindende Wandsegmente ohne Faltenbildung nach innen biegen können. Ferner kann die Einlage außen einen oder mehrere selbstklebende Bereiche aufweisen, die zur Befestigung in dem Büstenhaltercup dienen.

Im folgenden werden verschiedene Ausgestaltungen der Wirkstoffzonen bechrieben, die mit Eigenschaften zur Dosierung der Wirkstoffe ausgebildet sind. Insbesondere, wenn das Wirkstoffpräparat eine relativ hohe Viskosität aufweist, können die Wirkstoffzonen hygroskopisch ausgebildet sein, um beispielsweise durch Aufnahme von Körperfeuchtigkeit das Präparat allmählich zu verdünnen und zerfließen zu lassen. Eine weitere Möglichkeit der Dosierung der Wirkstoffe kann darin bestehen, daß ein Druckunterschied zwischen den Wirkstoffbereichen und der Haut vorgesehen wird, durch den Ionen oder Moleküle des Wirkstoffpräparats einen bestimmten Druck auf die Haut ausüben. Durch unterschiedliche Konzentration der Wirkstoffe kann ein Transport in Richtung Haut bewirkt werden (controlled release). Eine Anlagerung von bestimmten Ionen oder Molekülen an die Haut kann auch dadurch erreicht werden, daß eine elektrische Spannung mindestens über die Dicke der Wirkstoffzonen angelegt ist, z.B. mittels einer Folienbatterie, wodurch erreicht wird, daß sich ladungsabhängig bestimmte Ionen oder Moleküle an einer Elektrode anlagern, die an die Haut angrenzt und von dieser isoliert ist. Gemäß einer weiteren Ausgestaltung der Erfindung können die Wirkstoffzonen an der Innenseite durch eine diaphragmaartige Trennschicht begrenzt sein, so daß die Wirkstoffe osmotisch auf die Haut einwirken können. Das verwendete Wirkstoffpräparat kann ferner so beschaffen sein, daß sich die Wirkstoffe in einer Trägersubstanz befinden, die sich bei Erwärmung verflüssigt, oder die Wirkstoffe können selbst eine Substanz darstellen, die diese Eigenschaft aufweist. Die Erwärmung erfolgt dabei durch die Körpertemperatur. Es können auch Substanzen vorgesehen sein, die thixotrop sind. Eine weitere Möglichkeit zur Dosierung der Wirkstoffe besteht darin, die Wirkstoffzonen an ihrer Innenseite durch eine Membran zu begrenzen, deren Durchlässigkeit für die Wirkstoffe sich bei Erwärmung erhöht. Es kann auch eine Membran vorgesehen sein, deren Durchlässigkeit für die Wirkstoffe sich bei Krafteinwirkung durch Bewegungsänderungen der Einlage, also durch Körperbewegungen, erhöht. Die Wirkstoffe können auch grenzflächenaktive (kapillaraktive) Stoffe sein, die sich beispielsweise an den Grenzflächen zwischen den Wirkstoffzonen und der Haut anreichern. Die Wirkstoffzonen sind bei dieser Ausgestaltung als Grenzflächen-Systeme ausgebildet.

Die Erfindung wird anhand nachfolgender Zeichnungen näher erläutert. In den Zeichnungen zeigen:
- Fig. 1: eine perspektivische Ansicht einer größenverstellbaren Einlage;
- Fig. 2a und 2b: Seitenansichten von Einlagen mit streifenförmigen Wirkstoff- und Kompensationszonen;
- Fig. 3: eine perspektivische Ansicht einer Einlage mit Ausnehmungen und Wandsegmenten zur Größenanpassung der Einlage;
- Fig. 4: eine schematische Teilschnittansicht einer Anordnung von Wirkstoff- und Kompensationszonen und eines angrenzenden Büstenhaltermaterials;
- Fig. 5: eine Teilschnittansicht einer Wirkstoffzone mit einer angrenzenden Übertragungszone und einer angelegten elektrischen Spannung.

In allen Figuren sind gleiche Teile mit gleichen Bezugszeichen bezeichnet.

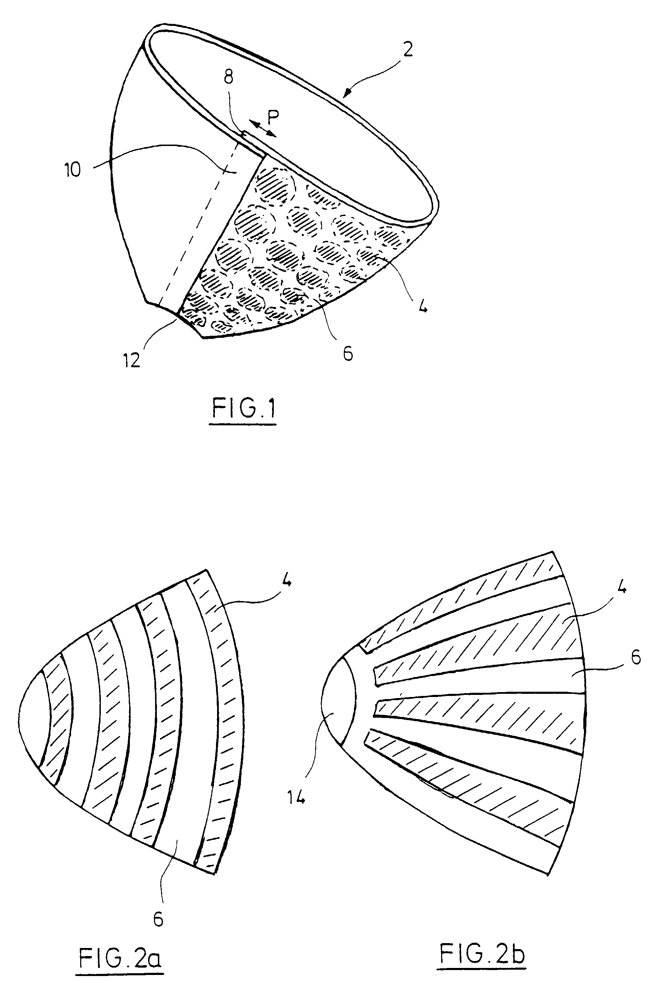

Fig. 1 zeigt eine Einlage 2 mit einer Vielzahl Wirkstoffzonen 4, die eine runde Umfangsform aufweisen. Die Wirkstoffzonen sind über die gesamte Fläche der Einlage angeordnet, allerdings für die linke Hälfte der Einlage aus Gründen der Übersichtlichkeit nicht gezeigt. Bereiche zwischen den Wirkstoffzonen bilden zusammen eine Kompensationszone 6. Die Einlage weist in Längsrichtung eine Trennlinie auf, und zwei Seitenbereiche 8, 10 entlang der Linie, die sich überlappen, wobei die Stärke der Überlappung, wie durch den doppelpfeil P angedeutet, einstellbar ist, um die Größe der Einlage der Größe des Cups anpassen zu können. Die Einlage weist an dem vorderen Ende eine Öffnung 12 auf, die dazu dient, die Brustwarze nicht mit der Einlage in Kontakt zu bringen.

Es ist aber auch möglich, daß die Zonen 4 gleichzeitig atmungsaktive Kompensationszonen sind.

Die Fig. 2a und 2b zeigen Einlagen mit streifenförmigen Wirkstoffzonen 4 und im wesentlichen streifenförmigen Kompensationszonen 6. Bei der Einlage der Fig. 2a sind die Streifen 4, 6 in Umfangsrichtung angeordnet, in Fig. 2b hingegen sind sie in Längsrichtung angeordnet. Beide Einlagen sind im Gegensatz zu der in Fig. 1 gezeigten Einlage an dem vorderen Ende geschlossen. Während die Einlage der Fig. 2a an dem vorderen Ende mit einer Kompensationszone ausgebildet ist, weist die Einlage der Fig. 2b an dem vorderen Ende weder eine Kompensationszone noch eine Wirkstoffzone auf. Das vordere Ende stellt eine weitere Zone einer "neutralen" Zone 14 innerhalb einer die Wirkstoffund die Kompensationszonen enthaltenden Lage dar.

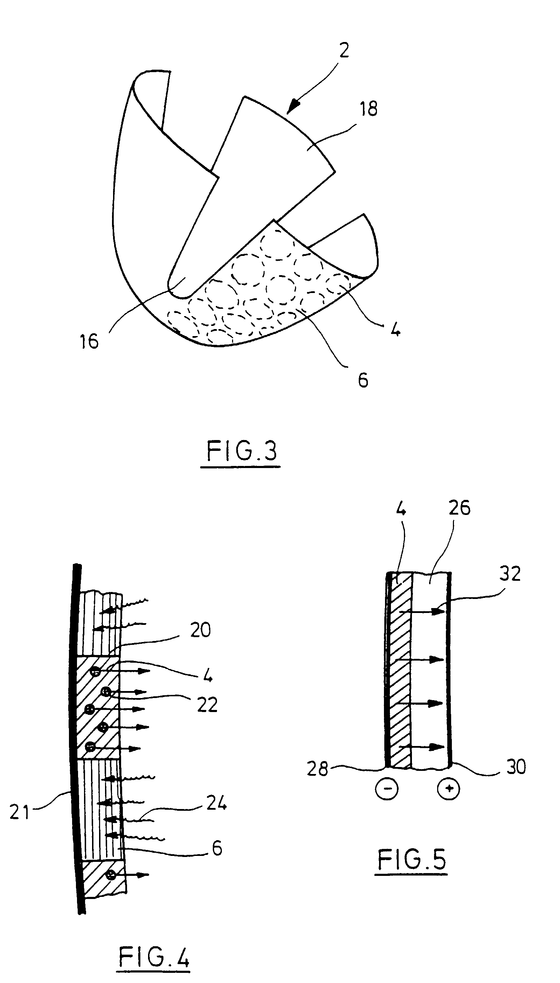

Fig. 3 zeigt eine Einlage mit Wirkstoffzonen 4 und Kompensationszonen 6, die wie bei der Einlage der Fig. 1 angeordnet sind. Zur Größenanpassung der Einlage 2 an die Größe eines Cups sind drei sich in Längsrichtung von der Randkante erstreckende Ausnehmungen 16 mit einer keilförmigen, sich zum vorderen Ende der Einlage hin verjüngenden und abgerundeten Form vorhanden. Durch diese Ausnehmungen wird die Einlage in drei Wandsegmente 18 unterteilt, die über das vordere Ende der Einlage miteinander verbunden sind und sich bei seitlichem Druck nach innen biegen können, wodurch sich der Umfang der Einlage ohne eine Faltenbildung verringern kann.

Fig. 4 erläutert das Funktionsprinzip der Wirkstoffzonen 4 und der Kompensationszonen 6. Bei der in Fig. 4 dargestellten Anordnung dieser beiden Zonentypen grenzen diese abwechselnd unmittelbar aneinander. Sie sind durch Grenzflächen 20 getrennt, die für das in der Wirkstoffzone vorhandene Wirkstoffpräparat nicht durchlässig sind. An einer Längsseite, die die Außenseite darstellt, liegen die Zonen an ein Büstenhaltermaterial an. Dieses Material ist so gewählt, daß Wirkstoffteilchen 22 nur durch die gegenüberliegende Längsseite, also nach innen zur Haut hin aus den Wirkstoffzonen austreten können, wie durch die geraden Pfeile angedeutet ist. In umgekehrter Richtung können durch dieselbe Längsseite von der Haut abgegebene Substanzen in die atmungsaktiven Kompensationszonen eintreten, wie durch die Pfeile 24 angedeutet ist.

Fig. 5 zeigt einen Teil einer Wirkstoffzone 4 und einen Teil einer innenseitig an dieser anliegenden Übertragungszone 26. Über die gesamte Breite dieser beiden Zonen ist eine elektrische Spannung so angelegt, daß die Außenseite der Wirkstoffzone 28 negativ und die Innenseite der Übertragungszone 30 positiv gepolt ist. Diese Spannung kann beispielsweise mit Hilfe einer Folienbatterie angelegt werden. Negative Wirkstoffionen oder -molküle wandern, wie durch die Pfeile 32 angedeutet ist, von der Wirkstoffzone in die Übertragungszone zum positiven Pol 30 hin.

## Patentansprüche

1. Einlage für einen Büstenhalter, die an die Innenform des Cups der Büstenhalter angepaßt ist, eine eigene Formstabilität aufweist und atmungsaktiv ist, dadurch gekennzeichnet, daß mindestens eine mit Wirkstoff gespeicherte und den Wirkstoff an die Haut abgebende Zone (4) und mindestens eine als atmungsaktiver und Körperflüssigkeit aufnehmender Bereich dienende Kompensationszone (6) vorgesehen sind, wobei die Gesamtfläche der Zonen (4, 6) im wesentlichen der Gesamtfläche der Einlage (2) entspricht.

2. Einlage nach Anspruch 1, dadurch gekennzeichnet, daß die Wirkstoff speichernde Zone gleichzeitig eine atmungsaktive Zone ist

3. Einlage nach Anspruch 1 oder 2, dadurch gekennzeichnet, daß eine Vielzahl von Wirkstoffzonen (4) und eine Vielzahl von Kompensationszonen (6) abwechselnd angeordnet sind.

4. Einlage nach Anspruch 1 oder 3, dadurch gekennzeichnet, daß eine Vielzahl von Zonen der einen Art, nämlich von Wirkstoffzonen (4) oder von Kompensationszonen (6), voneinander getrennt angeordnet sind und die Bereiche dazwischen eine Zone der anderen Art, d.h. eine Kompensationszone (6) bzw. eine Wirkstoffzone (4), bilden.

5. Einlage nach einem der vorhergehenden Ansprüche, dadurch gekennzeichnet, daß Zonen streifenförmig in Umfangsrichtung oder Längsrichtung angeordnet sind

6. Einlage nach Anspruch 5, dadurch gekennzeichnet, daß die getrennt voneinander angeordneten Zonen eine runde Umfangsform aufweisen

7. Einlage nach einem der vorhergehenden Anspruche, dadurch gekennzeichnet, daß die Einlage (2) mehrere Lagen aufweist, von denen eine die Wirkstoff- und die Kompensationszone (4, 6) enthält.

8. Einlage nach Anspruch 7, dadurch gekennzeichnet, daß die Einlage (2) eine außen an die die Wirkstoff- und die Kompensationszonen (4, 6) enthaltende Lage angrenzende Lage aufweist, die atmungsaktiv und zumindest in an die Wirkstoffzonen (4) anliegenden Bereichen flüssigkeitsundurchlässig ist

9. Einlage nach Anspruch 7 oder 8, dadurch gekennzeichnet, daß die Einlage (2) eine innen an die Wirkstoff- und die Kompensationszonen (4, 6) enthaltende Lage angrenzende Lage zur Ubertragung der Wirkstoffe (22) oder durch die Kompensationszonen aufzunehmender Substanzen aufweist.

10. Einlage nach einem der Ansprüche 7 bis 9, dadurch gekennzeichnet, daß die Einlage (2) als innerste Lage einen entfernbaren Schutzfilm aufweist

11. Einlage nach einem der vorhergehenden Ansprüche, dadurch gekennzeichnet. daß die Grenzflächen (20) zwischen aneinandergrenzenden Wirkstoff- und Kompensationszonen (4, 6) eine begrenzte Durchlässigkeit aufweisen.

12. Einlage nach einem der vorhergehenden Ansprüche, dadurch gekennzeichnet, daß die Einlage (2) aus flexiblem Material besteht.

13. Einlage nach einem der Ansprüche 1 bis 11, dadurch gekennzeichnet, daß sie zumindest an der Innenseite thermoplastisches Material aufweist, das bei Körperoberflächentemperatur sich an die Haut anpaßt und guten Hautkontakt gewährleistet

14. Einlage nach einem der vorhergehenden Ansprüche, dadurch gekennzeichnet, daß die Einlage (2) ein aufgerollter Flachstreifen mit einem selbstklebenden Verschluß ist

15. Einlage nach Anspruch 14, dadurch gekennzeichnet, daß der Flachstreifen Schwächungslinien zur Kürzung des Streifens aufweist

16. Einlage nach einem der vorhergehenden Ansprüche, dadurch gekennzeichnet. daß die Einlage (2) in Längsrichtung eine Trennlinie aufweist und zwei Seitenbereiche (8, 10) entlang der Linie sich einstellbar überlappen.

17. Einlage nach einem der vorhergehenden Ansprüche, dadurch gekennzeichnet, daß die Einlage (2) in Längsrichtung Ausnehmungen (16) aufweist.

18. Einlage nach einem der vorhergehenden Ansprüche, dadurch gekennzeichnet, daß die Einlage (2) außen mindestens einen selbstklebenden Bereich zur Befestigung an dem Büstenhalter aufweist

19. Einlage nach einem der vorhergehenden Ansprüche, dadurch gekennzeichnet, daß die Wirkstoffzonen (4) hygroskopisch sind

20. Einlage nach einem der vorhergehenden Ansprüche, dadurch gekennzeichnet, daß sie so aufgebaut ist, daß ein Druckunterschied zwischen den Wirkstoffzonen (4) und der Haut besteht

21. Einlage nach einem der vorhergehenden Ansprüche, dadurch gekennzeichnet, daß sie so aufgebaut und der Wirkstoff so eingebaut ist, daß der Wirkstofftransport zur Haut durch ein Konzentrationsgefalle bewirkt wird (controlle release).

22. Einlage nach einem der vorhergehenden Ansprüche, dadurch gekennzeichnet, daß eine Folienbatterie derart angeordnet ist, daß eine elektrische Spannung (28, 30) über die Dicke der Wirkstoffzonen (4) angelegt ist.

23. Einlage nach einem der vorhergehenden Ansprüche, dadurch gekennzeichnet, daß die Wirkstoffzonen (4) innenseitig durch eine diaphragmaartige Trennschicht begrenzt sind.

24. Einlage nach einem der vorhergehenden Ansprüche, dadurch gekennzeichnet, daß die Wirkstoffe (22) sich in einer Substanz befinden oder eine Substanz darstellen, die sich bei Erwärmung verflüssigt.

25. Einlage nach einem der vorhergehenden Ansprüche, dadurch gekennzeichnet, daß die Wirkstoffe (22) sich in einer Substanz befinden oder eine Substanz darstellen, die thixotrop ist.

26. Einlage nach einem der vorhergehenden Ansprüche, dadurch gekennzeichnet, daß die Wirkstoffzonen (4) innenseitig durch eine Membran begrenzt sind, deren Durchlässigkeit für die Wirkstoffe (22) sich bei Erwärmung erhöht.

27. Einlage nach einem der vorhergehenden Ansprüche, dadurch gekennzeichnet. daß die Wirkstoffzonen innenseitig durch eine Membran begrenzt sind, deren Durchlässigkeit für die Wirkstoffe (22) sich bei Krafteinwirkung durch Bewegungsänderungen der Einlage (2) erhöht.

28. Einlage nach einem der vorhergehenden Ansprüche, dadurch gekennzeichnet, daß die Wirkstoffzonen (4) Grenzflächensysteme mit grenzflächenaktiven Wirkstoffen (22) sind.

## Claims

1. An insert for a bra, which is adapted to the inner shape of the cup of the bra, has a dimensional stability of its own and is breathing, characterised in that the insert comprises at least one zone (4) storing an active ingredient and delivering the active ingredient to the skin, and at least one breathing compensation zone provided to take up body substances, wherein the total surface of the zones (4, 6) corresponds essentially to the total surface of the insert (2).

2. The insert according to claim 1, characterised in that the zone storing the active ingredient is at the same time a breathing zone.

3. The insert according to claim 1 or 2, characterised in that a multitude of active ingredient zones (4) and a multitude of compensation zones (6) are arranged alternately.

4. The insert according to claim 1 or 3, characterised in that a multitude of zones of the one type, that is of active ingredient zones (4) or of compensation zones (6) are arranged separated from one another and the regions there between form the zone of the other type, i.e. a compensation zone (6) or an active ingredient zone (4).

5. The insert according to one of the preceding claims, characterised in that zones are arranged strip-shaped in the circumferential direction or the longitudinal direction.

6. The insert according to claim 5, characterised in that the zones arranged separated from one another comprise a round circumferential shape.

7. The insert according to one of the preceding claims, characterised in that the insert (2) comprises several layers of which one contains the active ingredient and compensation zone (4, 6).

8. The insert according to claim 7, characterised in that the insert (2) comprises a layer, externally bordering the layer containing the active ingredient and compensation zones (4, 6), which is breathing and at least in the regions neighbouring the active ingredient zones (4) is permeable to fluid.

9. The insert according to claim 7 or 8, characterised in that the insert (2) comprises a layer, internally bordering the active ingredient and compensation zones (4, 6), which is for transmitting the active ingredients (22) or substances to be absorbed by the compensation zones.

10. The insert according to one of claims 7 to 9, characterised in that the insert (2) comprises a removable protective film as an innermost layer.

11. The insert according to one of the preceding claims, characterised in that the border surfaces (20) between active ingredient zones and compensation tones (4, 6) bordering one another have a limited permeability.

12. The insert according to one of the preceding claims, characterised in that the insert (2) consists of flexible material.

13. The insert according to one of claims 1 to 11, characterised in that it comprises thermoplastic material at least on the inner side, which with body surface temperature adapts to the skin and ensures a good skin contact.

14. The insert according to one of the preceding claims, characterised in that the insert (2) is a rolled up flat strip with a self-adhesing closure.

15. The insert according to claim 14, characterised in that the flat strip comprises weakening lines for shortening the strip.

16. The insert according to one of the preceding claims, characterised in that the insert (2) in the longitudinal direction comprises a separating line and two lateral regions (8, 10) adjustably overlap along the line.

17. The insert according to one of the preceding claims, characterised in that the insert (2) comprises recesses (16) in the longitudinal direction.

18. The insert according to one of the preceding claims, characterised in that the insert (2) externally comprises at least a self-adhesing region for fastening to the bra.

19. The insert according to one of the preceding claims, characterised in that the active ingredient zones (4) are hygroscopic.

20. The insert according to one of the preceding claims, characterised in that it is constructed such that there exists a pressure difference between the active ingredient zones (4) and the skin.

21. The insert according to one of the preceding claims, characterised in that it is so constructed and the active ingredient is so incorporated that the active ingredient transport to the skin is effected by falls in concentration (controlled release).

22. The insert according to one of the preceding claims, characterised in that a foil battery is arranged in such a manner that an electrical voltage (28, 30) is applied over the thickness of the active ingredient zones (4).

23. The insert according to one of the preceding claims, characterised in that the active ingredient zones (4) on the inner side are limited by a diaphragm-like separating layer.

24. The insert according to one of the preceding claims, characterised in that the active ingredients (22) are located in a substance or form a substance which liquefies on heating.

25. The insert according to one of the preceding claims, characterised in that the active ingredients (22) are located in a substance or form a substance which is thixotropic.

26. The insert according to one of the preceding claims, characterised in that the active ingredient zones (4) on the inside are limited by a membrane whose permeability to the active ingredients (22) increases on heating.

27. The insert according to one of the preceding claims, characterised in that the active ingredient zones on the inside are limited by a membrane whose permeability to the active ingredients (22) increases with the effect of force by changes in movement of the insert (2).

28. The insert according to one of the preceding claims, characterised in that the active ingredient zones (4) are border surface systems with border-surface-active active ingredients (22).

## Revendications

1. Garniture pour soutien-gorge, adaptée à la forme intérieure du bonnet du soutien-gorge, présentant une stabilité de forme propre et thermoactive, caractérisée en ce que sont prévues au moins une zone (4) remplie de matière active et distribuant la matière active à la peau et au moins une zone de compensation (6) servant de domaine thermoactif et absorbant un liquide corporel, la surface totale des zones (4, 6) correspondant pour l'essentiel à la surface totale de la garniture (2).

2. Garniture selon la revendication 1, caractérisée en ce que la zone stockant la matière active est simultanément une zone thermoactive.

3. Garniture selon la revendication 1 ou 2, caractérisée en ce qu'une multiplicité de zones (4) de matière active et une multiplicité de zones de compensation (6) sont disposées en alternance.

4. Garniture selon la revendication 1 ou 3, caractérisée en ce qu'une multiplicité de zones d'une des sortes, à savoir de zones (4) de matière active ou de zones de compensation (6), sont disposées séparées les unes des autres, les domaines compris entre elles formant une zone de l'autre sorte, c'est-à-dire respectivement une zone de compensation (6) ou une zone (4) de matière active.

5. Garniture selon l'une des revendications précédentes, caractérisée en ce que les zones sont disposées sous forme de bandes dans la direction périphérique ou la direction longitudinale.

6. Garniture selon la revendication 5, caractérisée en ce que les zones disposées séparées les unes des autres présentent une forme périphérique ronde.

7. Garniture selon l'une des revendications précédentes, caractérisée en ce que la garniture (2) comporte plusieurs couches dont l'une contient la zone de matière active et la zone de compensation (4, 6).

8. Garniture selon la revendication 7, caractérisée en ce que la garniture (2) comporte une couche adjacente extérieurement à la couche contenant les zones de matière active et de compensation (4, 6), qui est thermoactive et, au moins dans les domaines adjacents aux zones (4) de matière active, est imperméable aux liquides.

9. Garniture selon la revendication 7 ou 8, caractérisée en ce que la garniture (2) comporte une couche adjacente intérieurement à la couche contenant les zones de matière active et de compensation (4, 6) pour la transmission des matières actives (22) ou des substances à absorber par les zones de compensation.

10. Garniture selon l'une des revendications 7 à 9, caractérisée en ce que la garniture (2) comporte comme couche la plus intérieure un film de protection amovible.

11. Garniture selon l'une des revendications précédentes, caractérisée en ce que les surfaces limites (20) entre zones de matière active et de compensation (4, 6) adjacentes présentent une perméabilité limitée.

12. Garniture selon l'une des revendications précédentes, caractérisée en ce que la garniture (2) est composée d'un matériau flexible.

13. Garniture selon l'une des revendications 1 à 11, caractérisée en ce qu'elle comporte, au moins sur le côté intérieur, un matériau thermoplastique qui, à la température superficielle du corps, s'adapte à la peau et garantit un bon contact avec la peau.

14. Garniture selon l'une des revendications précédentes, caractérisée en ce que la garniture (2) est une bande plate enroulée pourvue d'une fermeture autoadhésive.

15. Garniture selon la revendication 14, caractérisée en ce que la bande plate comporte des lignes d'affaiblissement pour le raccourcissement de la bande.

16. Garniture selon l'une des revendications précédentes, caractérisée en ce que la garniture (2) comporte une ligne de séparation dans la direction longitudinale et en ce que deux domaines latéraux (8, 10) se recouvrent de manière réglable le long de ladite ligne.

17. Garniture selon l'une des revendications précédentes, caractérisée en ce que la garniture (2) comporte des évidements (16) dans la direction longitudinale.

18. Garniture selon l'une des revendications précédentes, caractérisée en ce que la garniture (2) comporte à l'extérieur au moins un domaine autoadhésif pour la fixation au soutien-gorge.

19. Garniture selon l'une des revendications précédentes, caractérisée en ce que les zones (4) de matière active sont hygroscopiques.

20. Garniture selon l'une des revendications précédentes, caractérisée en ce qu'elle est constituée de telle manière qu'il existe une différence de pression entre les zones (4) de matière active et la peau.

21. Garniture selon l'une des revendications précédentes, caractérisée en ce qu'elle est constituée de telle manière et la matière active est incorporée de telle manière que le transport de matière active vers la peau est effectué par un gradient de concentration (controlle release).

22. Garniture selon l'une des revendications précédentes, caractérisée en ce qu'une batterie en feuilles est disposée de telle manière qu'une tension électrique (28, 30) est appliquée sur l'épaisseur des zones (4) de matière active.

23. Garniture selon l'une des revendications précédentes, caractérisée en ce que les zones (4) de matière active sont limitées du côté intérieur par une couche de séparation en forme de diaphragme.

24. Garniture selon l'une des revendications précédentes, caractérisée en ce que les matières actives (22) se trouvent dans une substance ou représentent une substance qui se liquéfie à l'échauffement.

25. Garniture selon l'une des revendications précédentes, caractérisée en ce que les matières actives (22) se trouvent dans une substance ou représentent une substance qui est thixotrope.

26. Garniture selon l'une des revendications précédentes, caractérisée en ce que les zones (4) de matière active sont limitées du côté intérieur par une membrane dont la perméabilité pour les matières actives (22) augmente à l'échauffement.

27. Garniture selon l'une des revendications précédentes, caractérisée en ce que les zones de matière active sont limitées du côté intérieur par une membrane dont la perméabilité pour les matières actives (22) augmente lors de l'application d'une force par des changements de mouvement de la garniture (2).

28. Garniture selon l'une des revendications précédentes, caractérisée en ce que les zones (4) de matière active sont des systèmes de surfaces limites contenant des matières actives (22) actives sur les surfaces limites.
